# EUROPEAN PATENT SPECIFICATION

(11) **EP 0 659 726 B1**
(45) Date of publication and mention of the grant of the patent: **03.11.1999**
(21) Application number: 94203682.3
(22) Date of filing: 19.12.1994
(51) Int. Cl.: C07C 45/53, C07C 45/33, C07C 29/132, C07C 29/50, C07C 27/12

(54) **Process for preparing a cycloalkanone and/or a cycloalkanol**
Verfahren zur Herstellung von einem Cycloalkanon und/oder Cycloalkanol
Procédé pour la préparation d'une cycloalcanone et/ou d'un cycloalcanol

(30) Priority: 23.12.1993 BE 9301446
(43) Date of publication of application: 28.06.1995
(73) Proprietor: DSM N.V., 6411 TE Heerlen (NL)
(72) Inventor: Kragten, Ubaldus Franciscus, NL-6191 EK Beek (L.) (NL); Baur, Henricus Anna Christiaan, NL-6049 BE Herten (NL)

(56) References cited:
- EP-A- 0 004 105
- FR-A- 1 369 732
- FR-A- 2 140 088
- US-A- 5 206 441

## Description

The invention relates to a process for preparing a cyclo alkanone and/or a cyclo alkanol by oxidizing a cyclo alkane and/or cyclo alkene having from 4 to 18 C atoms with oxygen to form a cyclo alkylhydroperoxide, followed by decomposition of the cyclo alkylhydroperoxide formed in the presence of a catalyst which contains a metal compound immobilized on a carrier material.

A similar process is described in EP-A-96798. This publication describes the preparation of cyclohexanone and cyclohexanol, in which cyclohexane is first oxidized to a cyclohexylhydroperoxide-containing oxidation mixture and in which the cyclohexylhydroperoxide is subsequently decomposed into cyclohexanone and cyclohexanol. In that process, the decomposition is effected in the presence of a heterogeneous decomposition catalyst consisting of cobalt immobilized on a zeolite. This decomposition catalyst is claimed in EP-A-96798 to have a longer life and to be more resistant to acids and water than metal-on-carrier catalysts known in the art, such as cobalt on carbon as described in US-A-2851496.

A disadvantage of the above-mentioned process is that it employs a zeolite as carrier material, which zeolite is difficult to get. Another disadvantage is that with such a cobalt-on-zeolite decomposition catalyst a small amount of cobalt little by little dissolves in the reaction mixture, resulting in limited catalyst life. A further disadvantage is that the presence of a separate water phase during the decomposition of the alkylhydroperoxides further diminishes the catalyst activity by encouraging the cobalt to dissolve in the reaction mixture. The separate water phase thereby causes a significant decrease in the activity of the decomposition catalyst over a short period of time (e.g. a few hours). The separate water phase forms as a byproduct in the oxidation reaction of cyclohexane and in the decompostion reaction. The negative effect of a separate water phase on the life of a heterogeneous decomposition catalyst is also described in, for instance, US-A-4042630.

The object of the invention is to provide a process in which a readily obtainable decomposition catalyst is employed, which catalyst retains its activity in spite of the (possible) presence of water in the oxidation mixture.

This object is achieved in that an amount of a basic, aqueous solution is present in the oxidation mixture so that a separate water phase with a pH higher than 8.5 is present during the decomposition and in that the metal of the catalyst is chosen from the group comprising Mn, Fe, Co, Ni and Cu and the carrier material is an inorganic carrier material chosen from the group comprising TiO₂, ZrO₂, MnO₂ and carbon, the carrier does not carry aliphatic or aromatic amine groups or sulphide groups.

It has been found that when the decomposition is effected using the process of the invention the decomposition catalyst remains active for a long period of time. An additional advantage is that the decomposition of the alkylhydroperoxide proceeds rapidly. Another additional advantage is that in this process a high ratio of alkanone to alkanol is achieved. This is advantageous if the alkanone is the desired end product which is often the case. Furthermore, the process of the invention obviates the need to remove water from the oxidation mixture before or during the decomposition of the alkylhydroperoxide.

It is known, for instance from WO-A-9216487 and the aforementioned EP-A-96798, to treat an oxidation mixture with a basic aqueous solution prior to decomposition. However, with such a pretreatment no basic aqueous solution is intended to remain present with the oxidation mixture after the neutralization so that there is a separate water phase during the decomposition. That the basic water phase is separated out in the processes described in EP-A-96798 and WO-A-9216487 is evident since, if such separation were not effected, the zeolite-A- and silica carrier materials utilized in the examples of these patent specifications would dissolve in the water/oxidation mixture. It is explicitely stated in US-A-4238415, in which neutralization is also effected that the water phase is separated from the oxidation mixture prior to decomposition of the cyclohexylhydroperoxide.

It is known from, for instance, US-A-4238415, GB-A-1382849 and EP-A-92867 to carry out the homogeneously catalyzed decomposition of cyclohexylhydroperoxides in the presence of a basic aqueous solution. However, the homogeneously catalyzed decomposition is totally different from the heterogeneously catalyzed decomposition. As a consequence, known processes for homogeneously catalyzed decomposition cannot as a rule be advantageously used in a heterogeneously catalyzed decomposition.

The aforementioned US-A-4042630 also discloses a process aimed at effecting an alkylhydroperoxide in the presence of a stable, heterogeneous, chromium-containing decomposition catalyst. The formation of a separate water phase during the decomposition is regarded as being the cause of the diminishing catalyst activity. According to US-A-4042630, the formation of a separate water phase can be prevented during the decomposition of a cycloalkylhydroperoxide by continuously stripping the oxidation mixture with a gas, in which process the water concentration remains low. A disadvantage of this process, however, is that it requires a complex decomposition reactor enabling decomposition and stripping to take place simultaneously as well as an elaborate apparatus for recirculating the stripping gas. Another disadvantage is that, along with water, also cyclohexane and reaction products such as cyclohexanol and cyclohexanone are stripped from the oxidation mixture by the stripping gas, so that these valuable compounds need to be recovered.

The decomposition catalyst of the invention consists of a metal compound, usually a metal oxide compound, which metal compound is immobilized on a carrier material, which carrier material is stable in the presence of a separate water phase of pH higher than 8.5. The metal oxide compound is a metal oxide selected from the group consisting of Mn, Fe, Co, Ni and Cu. It has been found that metals that form a stable anion complex, such as chromium, are less suited for application in the process of the invention.

The metal oxide may be applied to the carrier by any process known to those skilled in the art. Such catalysts may for instance be prepared in a single step starting from readily available starting materials by applying methods often described in the literature such as impregnation and deposition-precipitation methods. Such methods are described in, for instance, J.W. Geus, Preparation of Catalysts III, pages 1 through 33, Studies in Surface Science and Catalsis Vol. 16, G. Poncelet, P. Grange and P.A. Jacobs eds., Elsevier 1983, and in P.J. v.d. Brink, A. Scholten, A. v. Wageningen, M.D.A. Lamers, A.J. v. Dillen, J.W. Geus, Preparation of Catalysts V pages 527 through 536, Studies in Surface Science and Catalysis Vol. 63, G. Poncelet, P.A. Jacobs, P. Grange and B. Delmon eds., Elsevier 1991. A metal compound for instance, which compound need not necessarily be the metal oxide, can be dissolved in a suitable solvent such as water and subsequently be contacted with the carrier and depostied on the carrier surface. Next, the carrier is calcined at a temperature higher than, say, 500°C, in which process the metal oxide is formed. Examples of suitable metal compounds include water-soluble metal compounds for example Co(OH)₂, Co(OH)₃, CuO, Cu₂O, Fe₂O₃, CoOₓ.yH₂O, CuOₓ.yH₂O, FeOₓ.yH₂O, MnOₓ.yH₂O and NiOₓ.yH₂O, where x may be equal to ½, 1, 1½, 2 or 3 and where y may have a value of from 0 to 20.

The carrier material may be any organic or inorganic carrier material that is stable in the presence of a separate water phase of pH higher than 8.5. Stable carrier materials will barely dissolve, if at all, in the reaction mixture during the decomposition. Preferably, the carrier material is an inorganic carrier material. The carrier material preferably has a hydrophilic surface so that a water layer will readily form around the catalyst during the decomposition. Examples of suitable carrier materials are TiO₂, ZrO₂, MnO₂ and carbon.

Another class of decomposition catalysts are based on Mn, Fe, Co, Ni and Cu with TiO₂ or ZrO₂ as carrier material to which a ligand is attached, there being no Si-O compounds present in the catalyst. Such catalysts are described in the afore mentioned WO-A-9216487. The metal compound and the ligands form a complex so that the metal compounds remain immobilized on the carrier material. A disadvantage of these catalysts, however, is that they must be prepared in a plurality of steps and the starting materials for these catalysts are less readily available. Accordingly, it is preferred to use a carrier material without ligands, with the metal compound being directly linked to the carrier material. As a rule, such catalysts can be prepared in a single step using starting materials that are readily available. A further disadvantage of using a catalyst as described in WO-A-921648 with ligands attached to the carrier material, is that relatively less alkanone is produced during the decomposition. Still another disadvantage is that the rate of the decomposition reaction is lower than when the catalyst using a carrier material without ligands is used.

As a rule, the weight percentage of metal in relation to the carrier material (referred to the metal only) is between 0.05 and 8 wt.%. Preferably, the weight percentage is higher than 0.2 and lower than 4 wt.%.

Another group of suitable decomposition catalysts are the metal oxides and the metal carbonates of Mn, Fe, Co, Ni and Cu. This group of catalysts is characterized in that the metal compound and the carrier material are identical.

Another suitable decomposition catalyst is an all-metal catalyst having a thin layer of the corresponding metal oxide deposited on the outside surface. An all-metal catalyst means a solid structure in the form of a catalyst particle, wherein the solid wholly or almost wholly consists of metal. An example of a suitable all-metal catalyst is a pellet of cobalt having a thin layer of cobalt oxide (CoO) on the outside surface (resulting from oxidation of cobalt).

The basic aqueous solution added to the oxidation mixture may be any aqueous solution that causes a separate water phase to form in the oxidation mixture which has a pH higher than 8.5 and is inert with respect to the catalyst. In general, ammoniacal aqueous solutions and aqueous solutions which contain amines are not inert with respect to the catalyst according to the invention and thus are less suited for use in the present invention. As a rule, the aqueous solution will contain a dissolved amount of alkali metal hydroxide, alkali metal carbonate and/or alkali metal phosphate. Examples of suitable alkali metals are sodium and potassium, these metals being readily available. It is preferred to use an aqueous solution in which alkali metal hydroxide and preferably alkali metal carbonate are dissolved inasmuch as these compounds can be recirculated in an easy manner as described in, for instance, GB-A-1398293. According to this process, the water phase is separated out after the decomposition and subsequently combusted at a temperature of 550-1200°C, yielding solid alkali metal carbonate. The alkali metal carbonate so obtained can be dissolved in water and used anew as the basic aqueous solution according to the invention. The solid alkali metal carbonate may optionally be hydrolyzed to the alkali metal hydroxide, which compound may also be used in the preparation of the basic aqueous solution according to the invention. Preferably, alkali metal carbonate solutions are started from inasmuch as the aforementioned hydrolysis to the alkali metal hydroxide can then be omitted.

Preferably, a portion of the basic aqueous solution will consist of the water phase separated out after the decomposition since by so doing a large amount of water phase can be created whilst the consumption of basic aqueous solution remains limited. The ratio of this returned amount of water and the amount of water eventually discharged may be between 50 and 0.

According to the invention, the pH of the separate water phase (measured at 25°C) is higher than 8.5. More preferably, the pH is higher than 9.5 and most preferably higher than 10. Where an aqueous alkali metal carbonate solution is employed, the pH will be lower than 11. It has been found that, if the pH is any higher, extremely little metal compound dissolves in the reaction mixture consisting of the water phase and the oxidation mixture.

As a rule, the weight ratio of the oxidation mixture and the water phase during the decomposition of the alkylhydroperoxide is between 200:1 and 1:20 and preferably between 100:1 and 1:1. It has been found that if relatively more water phase is present during the decomposition the reaction rate of the decomposition will increase.

In the process according to the invention the alkane and/or alkene is oxidized in the liquid phase in a manner known in the art, using for instance air, pure oxygen or a mixture of oxygen and an inert gas at temperatures of between 120 and 200°C, in particular of between 140 and 180°C, for, say, 5 minutes to 24 hours. In the process, an amount of, say, 1 to 50% of the alkane and/or alkene is converted, the amount may also be from 1 to 25%. The pressure at which this oxidation takes place is not critical and usually is between 0.4 and 5.0 MPa.

The alkane and/or alkene is preferably oxidized in the absence of substances promoting the decomposition of the alkylhydroperoxide formed, such as compounds of transition metals. Therefore it is preferred that the reactor used for the oxidation step has an inert inner wall, for instance an inner wall of passivated steel, aluminium, glass, enamel or another such material. If the use of an oxidation catalyst is opted for after all, the amount of transition metal should preferably be very small, for instance in the order of from 0.1 to 10 parts by weight per million. As oxidation catalyst use may be made of compounds of, for instance, cobalt, chromium, manganese, iron, nickel or copper or mixtures thereof.

The decomposition of the alkylhydroperoxide in the oxidation mixture is effected with the aid of the immobilized metal complexes according to the invention. The decomposition catalyst may be applied in a variety of ways. Since the said catalyst is immobilized on a carrier material, it is possible to use slurry reactors or, for instance, packed beds for converting the alkylhydroperoxide. The heat of reaction released in the decomposition reaction must be adequately collected and carried off to ensure good temperature control of the process. In particular, heat removal is readily accomplished when slurry reactors are used. Use of slurry reactors also allows for the desired temperature to be maintained during the decomposition by applying, for instance, reflux cooling for at least a portion of the heat to be carried off. This obviates the need to recirculate evaporated products, consequently producing a somewhat favourable effect on the desired product yield. In such a situation, the amount of decomposition catalyst to be applied is, for instance, from 5 to 250 ppm metal in reference to the oxidation mixture. Preferably, an amount of from 10 to 150 ppm is applied.

The process can also be carried out with advantage in a packed-bed reactor because of the relatively high catalyst concentration reached in it. The packed-bed reactor is particularly advantageous when alkylhydroperoxide mixtures with a relatively low concentration are used. The water phase and the oxidation mixture may be passed through the reactor in the same direction of flow (cocurrent) or in opposite directions of flow (countercurrent). Countercurrent operation is preferred because this enables the highest base concentration (high pH) to be attained at the lowest alkylhydroperoxide concentration so that a higher conversion can be obtained in process equipment of a given size.

As a rule, the catalyst particles that make up the decomposition catalyst, if applied in a packed bed, will have a diameter larger than 0.5 mm, because otherwise the pressure drop across the reactor will be too high. Preferably, the diameter is between 0.7 and 3 mm. The catalyst particles may be of any desired shape. Examples of possible shapes are spherules, bars and granules.

The temperature during the decomposition is in general in the range from 25 to 200°C, preferably between 50 and 120°C. Normally, the pressure at which the decomposition takes place is chosen somewhat lower than that at which the oxidation takes place. The decomposition may be effected in the presence of oxygen so that a higher alkanone/alkanol (K/A) ratio can be attained. The decomposition rate depends in part on the concentration of the transition metal on the carrier, the concentration of hydroperoxide and the temperature. As a rule the decomposition takes between 5 and 300 min. Preferably, the residence time of the reaction mixture in the decomposition reactor is kept between 15 and 120 minutes, but this is not critical. Those skilled in the art can establish through simple analyses whether any alkylhydroperoxide remains in a treated mixture.

After decomposition of the alkylhydroperoxide, the water phase can readily be separated from the decomposition mixture, for instance by phase separation. The separated water phase can be partly reused in a subsequent decomposition as earlier described herein. As a rule, the remaining amount of aqueous solution is disposed of or further processed. This waste stream largely consists of alkali metal salts with inorganic and/or organic acids.

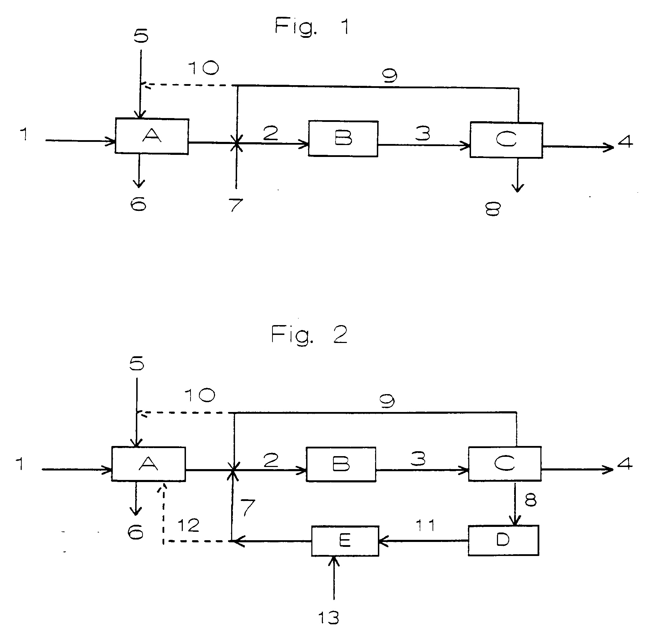

The addition of a basic aqueous solution may optionally be combined with a neutralization step and/or water wash as disclosed in the aforementioned WO-A-9216487 and EP-A-96798. Figure 1 shows schematically how a possible embodiment of the decomposition step according to the invention may be designed. The oxidation mixture obtained by oxidation of the alkane and/or alkene is supplied through stream (1) to an optional neutralization step A. There, the oxidation mixture is treated with, for instance, an aqueous alkali metal hydroxide or alkali metal carbonate solution (supplied through stream (5)), the carboxylic acids formed in the alkane and/or alkene oxidation being (partly) removed and/or neutralized. The water phase in step A can be discharged in whole or part through stream (6). Any water phase still present in the oxidation mixture in step A may either in its entirety or with addition of fresh basic aqueous solution supplied through stream (7) form the separate water phase according to the invention. Subsequently, the water/oxidation mixture is conveyed to the decomposition step B through stream (2). As a rule, after the decomposition step B, the water phase is separated from the oxidation mixture by phase separation in separation step C, the water phase being discharged through stream (8). A portion of the water phase is preferably reused and recirculated through stream (9) to stream (2) and/or to stream (5) through stream (10). The K/A mixture exits through stream (4). The oxidation mixture may optionally be subjected to a water wash before and after the above decomposition step and pretreatment step.

Figure 2 is a schematic representation of the embodiment of the decomposition step according to Figure 1 in which the alkali metal carbonate or alkali metal hydroxide is reused by combusting the discharged water phases as described hereinabove. The water phase is discharged to the combustion installation D through stream (8). The alkali metal carbonate that forms here is discharged through stream (11) and is subsequently dissolved in water (supplied through stream (13)). The basic aqueous solution so obtained may subsequently be recirculated through stream (7) and/or stream (12) to the optional neutralization step A.

After separation of the water phase, the reaction mixture obtained on decomposition of the hydroperoxide can be further processed by subjecting the organic phase to distillation with recovery of alkane and/or alkene as well as alkanol and alkanone to be returned to the oxidation reaction.

As alkane and/or alkene having from 3 to 30 C atoms use may be made of, for instance, propane, 2-methylpropane, cyclopheptane, cyclohexane, cyclooctane, cyclododecane and cyclohexene. The alkane and/or alkene may comprise aromatic groups and ethylenically unsaturated groups, for instance methylbenzene, ethylbenzene, 2-propylbenzene and phenylcyclohexane. The alkane and/or alkene may be branched, linear and/or cyclic.

The process is particularly suitable for oxidizing cycloalkanes having from 4 to 18, particularly from 6 to 12 carbon atoms, more particularly for oxidizing cyclohexane, cyclooctane and cyclododecane, it being possible to utilize the reaction products from the cyclohexane oxidation especially for either the preparation of caprolactam (for nylon 6) or the preparation of adipic acid (for nylon 6,6). The cyclohexanol and cyclohexanone so obtained have been found to be pure enough, without further treatment, for further conversion into suitable starting materials for the preparation of caprolactam.

The invention is elucidated by the following examples.

The selectivity for cycloalkanol and cycloalkanone is calculated by dividing the sum of the cycloalkanol and cycloalkanone formed during the decomposition by the amount of cycloalkyl hydroperoxide converted during the decomposition (amounts in moles).

### Example I

This example describes the preparation of Type A catalyst. An aqueous solution of ammonium cobalt (II) EDTA (ethylene diamine tetraacetic acid) complex was prepared by dissolving Co(NO₃)₂.6H₂O and EDTA at a molar ratio of 1/1 in water of high purity and then adding 25-wt.% NH₃ water solution so that ultimately the pH was equal to 9.0. The cobalt concentration of the resulting solution was 0.58 mol/l. This resulting solution was used for impregnating a TiO₂ carrier material, with 10 g of TiO₂ extrudates (Norton Chemical Process Products Corporation; BET surface area 172 m²/g, pore volume 0.31 cm³/g, 3 mm cross-sectional area, length 1 cm). The carrier material was kept under vacuum for 10 minutes. Subsequently, 9.5 cm³ of the cobalt solution was added to the carrier material while maintaining the vacuum. The amount of cobalt was sufficient for conducting full wetting of the carrier material. Next, the extrudates were vacuum-dried; for one hour at 30°C, then for one hour at 50°C, then for one hour at 70°C, then for one hour at 90°C and then for one hour at 100°C. Finally, the catalyst particles were calcined in an electric furnace for 4 hours at (a maximum temperature) of 500°C, the temperature in the oven being increased in increments of 1°C per minute.

The calcined catalyst was brown and, on crushing some granules, the fragments appeared to be brown also, which is indicative of a homogeneous cobalt distribution.

The cobalt content was determined through neutron activation analysis (NAA) to be 2.6 wt.%.

### Example II

Example I was repeated to prepare the catalysts listed in Table I below.

**TABLE I**

| Type | Carrier | Metal | Metal content (wt.%) |
|---|---|---|---|
| A | TiO₂ | Co | 2.6 |
| B | ZrO₂ | Co | 1.1 |
| C | TiO₂ | Fe | 3.5 |
| D | TiO₂ | Cu | 3.0 |
| E | TiO₂ | Ni | 2.5 |
| F | TiO₂ | Mn | 1.9 |

### Batch experiments

### Example III

9.9 g of water phase in which Na₂CO₃ (750 mmol Na₂CO₃/kg) was added at room temperature to 99 g of a cyclohexane oxidation mixture containing 200 mmol cyclohexylhydroperoxide (CHHP) per kilogram, 60 mmol cyclohexanol (OL) per kilogram and 30 mmol cyclohexanone (ONE) per kilogram.
This mixture was heated to a temperature of 73°C, whereupon 0.626 g of Type A catalyst (cat.) prepared in accordance with Example I was added. The decomposition of CHHP was monitored by means of iodometric titration. The first-order rate constant was 4.2 * 10⁻⁴ kg solution/(g cat * min). The selectivity for cyclohexanol plus cyclohexanone was 103.3%. The molar cyclohexanol/cyclohexanone ratio was 0.74.

### Example IV

Example III was repeated except that 49 g of water phase in which Na₂CO₃ (750 mmol Na₂CO₃/kg water) was dissolved was added. This mixture was heated to a temperature of 73°C, whereupon 0.68 g of catalyst (Type A) was added. The decomposition of the CHHP was monitored by means of iodometric titration. The first-order rate constant was 9.5 * 10⁻⁴ kg solution/(g cat * min). The selectivity for cyclohexanol plus cyclohexanone was 100.6%. The molar cyclohexanol/cyclohexanone ratio was 0.64.

### Example V

Example III was repeated except that 200 g of water phase in which Na₂CO₃ (750 mmol/kg) was dissolved was added. This mixture was heated to a temperature of 73°C, whereupon 0.64 g of catalyst (Type A) was added. The decomposition of the CHHP was monitored by means of iodometric titration. The first-order rate constant was 22.6 * 10⁻⁴ kg solution/(g cat * min).

### Example VI

Example III was repeated except that 10.4 g of water phase in which Na₂CO₃ (2000 mmol/kg) was dissolved was added. This mixture was heated to a temperature of 73°C, whereupon 0.60 g of catalyst (Type A) was added. The decomposition of the CHHP was monitored by means of iodometric titration. The first-order rate constant was 13.0 * 10⁻⁴ kg solution/(g cat * min). The selectivity for cyclohexanol plus cyclohexanone was 97.3%. The molar cyclohexanol/cyclohexanone ratio was 0.71.

### Example VII

Example III was repeated except that 11.0 g of water phase in which Na₂CO₃ (1000 mmol/kg) was dissolved was added. This mixture was heated to a temperature of 73°C, whereupon 0.64 g of catalyst (Type B) was added. The decomposition of the CHHP was monitored by means of iodometric titration. The first-order rate constant was 10.2 * 10⁻⁴ kg solution/(g cat * min). The selectivity for cyclohexanol plus cyclohexanone was 100.3%. The molar cyclohexanol/cyclohexanone ratio was 0.74.

### Example VIII

Example III was repeated except that 11.2 g of water phase in which Na₂CO₃ (1000 mmol/kg) was dissolved was added. This mixture was heated to a temperature of 73°C, whereupon 0.64 g of catalyst (Type C) was added. The decomposition of the CHHP was monitored by means of iodometric titration. The first-order rate constant was 1.25 * 10⁻⁴ kg solution/(g cat * min).

### Example IX

Example III was repeated except that 11.2 g of water phase in which Na₂CO₃ (1000 mmol/kg) was dissolved was added. This mixture was heated to a temperature of 73°C, whereupon 0.84 g of catalyst (Type D) was added. The decomposition of the CHHP was monitored by means of iodometric titration. The first-order rate constant was 2.02 * 10⁻⁴ kg solution/(g cat * min).

### Example X

Example III was repeated except that 10.2 g of water phase in which Na₂CO₃ (1000 mmol/kg) was dissolved was added. This mixture was heated to a temperature of 73°C, whereupon 0.98 g of catalyst (Type E) was added. The decomposition of the CHHP was monitored by means of iodometric titration. The first-order rate constant was 1.2 * 10⁻⁴ kg solution/(g cat * min).

### Example XI

Example III was repeated except that 10.4 g of water phase in which Na₂CO₃ (1000 mmol/kg) was dissolved was added. This mixture was heated to a temperature of 73°C, whereupon 0.97 g of catalyst (Type F) was added. The decomposition of the CHHP was monitored by means of iodometric titration. The first-order rate constant was 1.6 * 10⁻⁴ kg solution/(g cat * min).

### Example XII

Example III was repeated except that 78 g of water phase in which Na₂CO₃ (1000 mmol/kg) was dissolved was added. This mixture was heated to a temperature of 73°C, whereupon 0.82 g of an all-cobalt catalyst (pellets 5 mm in diameter) was added. The decomposition of the CHHP was monitored by means of iodometric titration. The first-order rate constant was 8.5 * 10⁻⁴ kg solution/(g cat * min).

### Example XIII

Example III was repeated except that 10.2 g of water phase in which Na₂CO₃ (750 mmol/kg) was dissolved was added. This mixture was heated to a temperature of 73°C, whereupon 0.84 g of pelletized CoCO₃ (pellets 1 cm in diameter) was added. The decomposition of the CHHP was monitored by means of iodometric titration. The first-order rate constant was 46.2 * 10⁻⁴ kg solution/(g cat * min).

### Example XIV

Example III was repeated except that 10.4 g of water phase in which NaOH (2000 mmol/kg) was dissolved was added. This mixture was heated to a temperature of 73°C, whereupon 0.64 g of catalyst (Type A) was added. The decomposition of the CHHP was monitored by means of iodometric titration. The first-order rate constant was 15.7 * 10⁻⁴ kg solution/(g cat * min).

### Example XV

Example III was repeated except that 10.4 g of water phase in which K₂CO₃ (1000 mmol/kg) was dissolved was added. This mixture was heated to a temperature of 73°C, whereupon 0.77 g of a modified Type A catalyst containing 1.4 wt.% Co was added. The decomposition of the CHHP was monitored by means of iodometric titration. The first-order rate constant was 11.0 * 10⁻⁴ kg solution/(g cat * min).

### Example XVI

Example III was repeated except that 10 g of water phase in which RbOH (1000 mmol/kg) was dissolved was added. This mixture was heated to a temperature of 73°C, whereupon 0.64 g of catalyst was added. The decomposition of the CHHP was monitored by means of iodometric titration. The first-order rate constant was 20.4 * 10⁻⁴ kg solution/(g cat * min).

### Comparative Experiment A

Example III was repeated except that no water phase was added. On heating to a temperature of 73°C, 1.1 g of catalyst (Type A) was added. The decomposition of CHHP was monitored by means of iodometric titration. The first-order rate constant was 0.6 * 10⁻⁴ kg solution/(g cat*min).

### Example XVII

12 g of a water phase in which Na₂CO₃ (750 mmol/kg) was dissolved was added at room temperature to 100 g of a cyclododecane oxidation mixture containing 400 mmol cyclododecyl hydroperoxide (CDHP) per kilogram, 70 mmol cyclododecanol (DOL) per kilogram and 40 mmol cyclododecanone (DON) per kilogram. This mixture was heated to a temperature of 73°C, whereupon 0.7 g of a Type A catalyst with 2.4% Co was added. The decomposition of the CHHP was monitored by means of iodometric titration. The first-order rate constant was 3.6 * 10⁻⁴ kg solution/(g cat * min). The selectivity for DOL plus DON was 106.2%. The molar DOL/DON ratio was 0.85.

### Continuous experiments

### Example XVIII

6.0 g of a Type A catalyst was introduced into a hollow, glass container provided with slits of 1 mm. This container was placed in a 1-1 continuous-flow reactor provided with baffles. 45 g per hour of an organic phase (cyclohexane oxidation mixture containing 200 mmol cyclohexylhydroperoxide (CHHP) per kilogram, 60 mmol cyclohexanol (OL) per kilogram and 30 mmol cyclohexanone (ONE) per kilogram) and 13 g of a water phase in which Na₂CO₃ (1000 mmol/kg) was dissolved was added using two pumps. The two phases were mixed in the reactor with the aid of a turbine stirrer (1400 revolutions per min). The reaction temperature was 69°C. Any evaporating liquid was returned to the reactor by way of a reflux condenser. The liquid that overflowed was collected in a 5-l vessel. The conversion of the CHHP was determined by means of iodometric titration of this overflowing liquid. By operating this system in the manner as described herein, a CHHP conversion of 40 % was achieved over a period of 1000 hours. The conversion could be varied by varying the residence time, the amount of catalyst, the concentration of the base and the ratio of the water phase to the organic phase. The measured Co concentration in the effluent was less than 2 ppb.

### Comparative Experiment B

Example XVIII was repeated except that only water was added as the water phase. Acids which were present in the organic phase or otherwise formed were extracted towards the water phase, causing the pH in the water phase to drop below 7. The catalyst was completely deactivated within 24 hours.

### Comparative Experiment C

Comparative Experiment B was repeated except that as catalyst use was made of an aminosilane modified silica to which a Co salt was linked as described in Example XXVIII of WO-A-9216487. The catalyst was completely deactivated within 24 hours.

### Example XIX

15 g of catalyst (Type B) was introduced into a hollow, glass containers (sic) provided with slits of 1 mm. These containers (sic) were placed in a 1-l continuous-flow reactor provided with baffles. 45 g per hour of an organic phase (a cyclododecane oxidation mixture containing 400 mmol cyclododecylhydroperoxide (CDHP) per kilogram, 70 mmol cyclododecanol (DOL) per kilogram and 40 mmol cyclododecanone (DON) per kilogram) and 13 g of a water phase in which Na₂CO₃ (1000 mmol/kg) was dissolved was added using two pumps. The two phases were mixed in the reactor with the aid of a turbine stirrer (1400 revolutions per min). The reaction temperature was 89°C. Any evaporating liquid was returned to the reactor by way of a heated reflux condenser. The liquid that overflowed was collected in a 5-l vessel. The conversion of the CHHP was determined by iodometric titration of this overflowing liquid. By operating this system in the manner as described, a CDHP conversion of 80 % was achieved over a period of 500 hours. The conversion could be varied by varying the residence time, the amount of catalyst, the concentration of the base and the ratio of the water phase to the organic phase. The measured Co concentration in the effluent was less than 1 ppb.

## Claims

1. Process for preparing a cycloalkanone and/or a cycloalkanol by oxidizing a cycloalkane and/or cycloalkene having from 4 to 18 C atoms with oxygen to form a cycloalkylhydroperoxide, followed by decomposition of the a cycloalkylhydroperoxide formed in the presence of a catalyst which contains a metal compound immobilized on a carrier material, characterized in that during the decomposition a separate water phase with a pH higher than 8.5 is present and in that the metal of the catalyst is chosen from the group comprising Mn, Fe, Co, Ni and Cu and the carrier material is an inorganic carrier material chosen from the group comprising TiO₂, ZrO₂, MnO₂ and carbon, the carrier does not carry aliphatic or aromatic amine groups or sulphide groups.

2. Process according to claim 1, characterized in that the weight percentage of metal relative to the carrier material is between 0.2 and 4 wt.%.

3. Process according to any one of claims 1-2, characterized in that the basic aqueous solution is a solution which contains alkali metal hydroxide, alkali metal carbonate and/or alkali metal phosphate.

4. Process according to any one of claims 1-3, characterized in that the weight ratio of oxidation mixture and water phase is between 100:1 and 1:1.

5. Process according to any one of claims 1-4, characterized in that the pH of the water phase is higher than 9.

6. Process according to any one of claims 1-5, characterized in that the decomposition is effected in a packed-bed reactor and that the catalyst consists of particles with a particle size of between 0.7 and 3 mm.

7. Process according to any one of claims 1-6, characterized in that the cycloalkane has from 6 to 12 carbon atoms.

8. Process according to claim 7, characterized in that the cycloalkane is a cyclohexane.

9. Process according to any one of claims 1-8, characterized in that the basic water phase contains dissolved sodium carbonate.

10. Process according to any one of claims 1-9, characterized in that in a continuous process the water phase is separated out after the decomposition step and partly recirculated to the decomposition step.

11. Process according to any one of claims 1-10, characterized in that the basic water phase contains an alkali metal carbonate, the water phase being combusted after the decomposition at a temperature of from 550 to 1200°C, solid alkali metal carbonate being obtained, which alkali metal carbonate is reused for preparing the basic aqueous solution.

## Patentansprüche

1. Verfahren zur Herstellung eines Cycloalkanons und/oder eines Cycloalkanols durch Oxidieren eines Cycloalkans und/oder Cycloalkens, das 4 bis 18 Kohlenstoffatome aufweist, mit Sauerstoff zu einem Cycloalkylhydroperoxid, gefolgt von Zersetzung des gebildeten Cycloalkylhydroperoxids in Gegenwart eines Katalysators, der eine auf einem Trägermaterial immobilisierte Metallverbindung enthält, dadurch gekennzeichnet, daß während der Zersetzung eine getrennte Wasserphase mit einem pH-Wert höher als 8,5 vorliegt und daß das Metall des Katalysators ausgewählt ist aus der Gruppe, umfassend Mn, Fe, Co, Ni und Cu, und das Trägermaterial ein anorganisches Trägermaterial darstellt, ausgewählt aus der Gruppe, umfassend TiO₂, ZrO₂, MnO₂ und Kohlenstoff, wobei der Träger keine aliphatischen oder aromatischen Aminogruppen oder Sulfidgruppen trägt.

2. Verfahren nach Anspruch 1, dadurch gekennzeichnet, daß der Gewichtsprozentsatz an Metall, bezogen auf das Trägermaterial, zwischen 0,2 und 4 Gew.% liegt.

3. Verfahren nach einem der Ansprüche 1-2, dadurch gekennzeichnet, daß die wässerige basische Lösung eine Lösung darstellt, die Alkalimetallhydroxid, Alkalimetallcarbonat und/oder Alkalimetallphosphat enthält.

4. Verfahren nach einem der Ansprüche 1-3, dadurch gekennzeichnet, daß das Gewichtsverhältnis des Oxidationsgemisches und der Wasserphase zwischen 100:1 und 1:1 liegt.

5. Verfahren nach einem der Ansprüche 1-4, dadurch gekennzeichnet, daß der pH-Wert der Wasserphase höher als 9 ist.

6. Verfahren nach einem der Ansprüche 1-5, dadurch gekennzeichnet, daß die Zersetzung in einem Festbettreaktor bewirkt wird und daß der Katalysator aus Teilchen mit einer Teilchengröße zwischen 0,7 und 3 mm besteht.

7. Verfahren nach einem der Ansprüche 1-6, dadurch gekennzeichnet, daß das Cycloalkan 6 bis 12 Kohlenstoffatome aufweist.

8. Verfahren nach Anspruch 7, dadurch gekennzeichnet, daß das Cycloalkan Cyclohexan ist.

9. Verfahren nach einem der Ansprüche 1-8, dadurch gekennzeichnet, daß die basische Wasserphase gelöstes Natriumcarbonat enthält.

10. Verfahren nach einem der Ansprüche 1-9, dadurch gekennzeichnet, daß in einem kontinuierlichen Verfahren die Wasserphase nach dem Zersetzungsschritt abgetrennt wird und teilweise zu dem Zersetzungsschritt zurückgeleitet wird.

11. Verfahren nach einem der Ansprüche 1-10, dadurch gekennzeichnet, daß die basische Wasserphase ein Alkalimetallcarbonat enthält, wobei die Wasserphase nach der Zersetzung bei einer Temperatur von 550 bis 1200°C verbrannt wird, wodurch festes Alkalimetallcarbonat erhalten wird, wobei das Alkalimetallcarbonat zur Herstellung der wässerigen basischen Lösung wiederverwendet wird.

## Revendications

1. Procédé de préparation d'une cycloalcanone et/ou d'un cycloalcanol par oxydation d'un cycloalcane et/ou d'un cycloalcène ayant de 4 à 18 atomes de carbone à l'aide d'oxygène pour former un hydroperoxyde de cycloalkyle, suivie de la décomposition de l'hydroperoxyde de cycloalkyle formé en présence d'un catalyseur qui contient un composé métallique immobilisé sur un support, caractérisé en ce qu'au cours de la décomposition, une phase aqueuse distincte avec un pH supérieure à 8,5 est présente et en ce que le métal du catalyseur est choisi dans le groupe comprenant Mn, Fe, Co, Ni et Cu et le support est un support minéral choisi dans le groupe comprenant TiO₂, ZrO₂, MnO₂ et le carbone, le support ne portant pas de groupes sulfures ou de groupes amines aliphatiques ou aromatiques.

2. Procédé selon la revendication 1, caractérisé en ce que le pourcentage pondéral du métal par rapport au support est compris en 0,2 et 4 %, en poids.

3. Procédé selon la revendication 1 ou 2, caractérisé en ce que la solution aqueuse basique est une solution qui contient un hydroxyde de métal alcalin, un carbonate de métal alcalin et/ou un phosphate de métal alcalin.

4. Procédé selon l'une quelconque des revendications 1 à 3, caractérisé en ce que le rapport pondéral du mélange d'oxydation et de la phase aqueuse est compris entre 100:1 et 1:1.

5. Procédé selon l'une quelconque des revendications 1 à 4, caractérisé en ce que le pH de la phase aqueuse est supérieur à 9.

6. Procédé selon l'une quelconque des revendications 1 à 5, caractérisé en ce que la décomposition s'effectue dans un réacteur à lit tassé et le catalyseur se compose de particules ayant une grosseur de particules comprise entre 0,7 et 3 mm.

7. Procédé selon l'une quelconque des revendications 1 à 6, caractérisé en ce que le cycloalcane possède de 6 à 12 atomes de carbone.

8. Procédé selon la revendication 7, caractérisé en ce que le cycloalcane est un cyclohexane.

9. Procédé selon l'une quelconque des revendications 1 à 8, caractérisé en ce que la phase aqueuse basique contient du carbonate de sodium dissous.

10. Procédé selon l'une quelconque des revendications 1 à 9, caractérisé en ce que dans un procédé continu, la phase aqueuse est séparée après l'étape de décomposition et partiellement recyclée vers l'étape de décomposition.

11. Procédé selon l'une quelconque des revendications 1 à 10, caractérisé en ce que la phase aqueuse basique contient un carbonate de métal alcalin, la phase aqueuse étant brûlée après la décomposition à une température de 550 à 1200°C, du carbonate de métal alcalin solide étant obtenu, lequel carbonate de métal alcalin est réutilisé pour la préparation de la solution aqueuse basique.
